(19) **Europäisches Patentamt**
**European Patent Office**
**Office européen des brevets**

(11) **EP 4 765 152 A1**

(12) **EUROPEAN PATENT APPLICATION**
published in accordance with Art. 153(4) EPC

(43) Date of publication:
**24.06.2026 Bulletin 2026/26**

(21) Application number: **24856735.6**

(22) Date of filing: **14.08.2024**

(51) International Patent Classification (IPC):
*G16H 30/40* (2018.01)     *G16H 30/20* (2018.01)
*A61B 5/00* (2006.01)     *A61B 5/353* (2021.01)
*G06V 10/25* (2022.01)     *G06V 10/46* (2022.01)
*G06V 10/764* (2022.01)     *G06T 7/11* (2017.01)
*G06T 7/62* (2017.01)

(52) Cooperative Patent Classification (CPC):
**A61B 5/00; A61B 5/353; G06T 7/11; G06T 7/62;
G06V 10/25; G06V 10/46; G06V 10/764;
G16H 30/20; G16H 30/40**

(86) International application number:
**PCT/KR2024/012101**

(87) International publication number:
**WO 2025/042123 (27.02.2025 Gazette 2025/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.08.2023   KR 20230108307
12.03.2024   KR 20240034517**

(71) Applicant: **Medipixel, Inc.**
**Seoul 06174 (KR)**

(72) Inventors:
• **KIM, Hyun-woo**
  **Seoul 06174 (KR)**
• **NOH, Soon Cheol**
  **Seoul 06174 (KR)**
• **MOK, Yeong-Heon**
  **Seoul 06174 (KR)**
• **WON, Donghyun**
  **Seoul 06174 (KR)**

(74) Representative: **Qip Patent & Recht**
**Dr. Kuehn & Partner mbB**
**Bavariaring 10**
**80336 München (DE)**

(54) **METHOD AND APPARATUS FOR MATCHING MEDICAL IMAGES**

(57)     This electronic apparatus may comprise: a video obtainment unit that obtains a first blood vessel video and a second blood vessel video obtained by recording a target blood vessel at a plurality of positions; and a processor that, for each of the first blood vessel video and the second blood vessel video, selects, from among a plurality of frame images included in a corresponding blood vessel video, a reference frame image of the corresponding blood vessel video by using a score for the analyzability of the target blood vessel shown in each frame image, and determines, based on information associated with a cardiac cycle of each reference frame image, a correspondence relationship between a reference frame image of the first blood vessel video and a reference frame image of the second blood vessel video.

**Start**

Obtain first blood vessel video and second blood vessel video
by capturing target blood vessel at plurality of positions — 410

For each of first blood vessel video and second blood vessel video, select,
from among plurality of frame images included in corresponding
blood vessel video, reference frame image of corresponding blood vessel video by
using score for analyzability of target blood vessel shown in each frame image — 420

Determine, based on information associated with cardiac cycle of each reference
frame image, correspondence relationship between reference frame image of
first blood vessel video and reference frame image of second blood vessel video — 430

**End**

**FIG. 4**

EP 4 765 152 A1

**Description**

**TECHNICAL FIELD**

Hereinafter, a technique for matching medical images in consideration of a cardiac phase is provided.

**BACKGROUND ART**

Angiography is a diagnostic procedure that visualizes blood vessels and their conditions using X-rays, and is a useful tool for examining vascular diseases. In angiography, which is useful for diagnosing vascular diseases, accurately identifying the size and shape of blood vessels is important for determining the severity of a disease and selecting an appropriate treatment method. In particular, in relation to selecting a treatment method, it is required to perform quantitative analysis on blood vessels and accurately calculate an index related to blood flow, and for this purpose, it is necessary to determine an angiography image suitable for analysis.

The above description has been possessed or acquired by the inventor(s) in the course of conceiving the present disclosure and is not necessarily an art publicly known before the present application is filed.

**DISCLOSURE OF THE INVENTION**

**TECHNICAL SOLUTIONS**

A method of processing a medical image, performed by an electronic apparatus, may include obtaining a first blood vessel video and a second blood vessel video by capturing a target blood vessel at a plurality of positions, for each of the first blood vessel video and the second blood vessel video, selecting, from among a plurality of frame images included in the corresponding blood vessel video, a reference frame image of the corresponding blood vessel video by using a score for analyzability of the target blood vessel shown in each frame image, and determining a correspondence relationship between the reference frame image of the first blood vessel video and the reference frame image of the second blood vessel video based on information associated with a cardiac cycle of each reference frame image.

The selecting of the reference frame image may include selecting candidate frame images from among the plurality of frame images in order of highest scores based on scores of the plurality of frame images, obtaining verified frame images by verifying the selected candidate frame images based on a type of the target blood vessel, and selecting the reference frame image from among the verified frame images.

The selecting of the candidate frame images may include detecting a region of interest (ROI) from each frame image based on information regarding a capturing angle of the corresponding frame image, and determining the score for a morphologic feature of the target blood vessel included in the region of interest of each frame image.

The method may further include, upon obtaining at least one blood vessel video of the first blood vessel video or the second blood vessel video, determining a type of the target blood vessel shown in the obtained at least one blood vessel video.

The obtaining of the verified frame images may include determining a type of the target blood vessel shown in each candidate frame image based on a blood vessel region corresponding to the target blood vessel segmented from the corresponding candidate frame image, and when a verification region mapped to the type of the target blood vessel includes at least a portion of the blood vessel region, restricting the corresponding candidate frame image from being selected as the verified frame images.

The selecting of the reference frame image from among the verified frame images may further include determining a physical size of the target blood vessel shown in each verified frame image based on a blood vessel region corresponding to the target blood vessel segmented from the corresponding verified frame image and a calibration factor, and selecting a reference frame image from among the verified frame images based on the physical size of the target blood vessel.

The determining of the correspondence relationship may include selecting, based on a physical size of the target blood vessel shown in second reference frame images of the second blood vessel video, a correspondence candidate of a first reference frame image of the first blood vessel video from among the second reference frame images, and matching, from among the selected correspondence candidates, a second reference frame image having a cardiac phase closest to a cardiac phase of the first reference frame image to the first reference frame image.

The selecting of the correspondence candidate may include, for each of the second reference frame images, when a ratio of a second physical size of the target blood vessel shown in the corresponding second reference frame image to a first physical size of the target blood vessel shown in the first reference frame image is less than a threshold ratio, excluding the corresponding second reference frame image from the correspondence candidate, and when the ratio of the physical size of the target blood vessel shown in the corresponding second reference frame image to the first physical size is greater than or equal to the threshold ratio, selecting the corresponding second reference frame image as the correspondence candidate.

The selecting of the correspondence candidate may include, for all of the second reference frame images, when a ratio of each second physical size to a first physical size of the target blood vessel shown in the first reference frame image is less than a threshold ratio, selecting all of the second reference frame images as correspondence candidates.

The matching may include, when a score of the second reference frame image having the closest cardiac phase is less than a threshold score, canceling selection of the first reference frame image, and adding at least one frame image from among the plurality of frame images of the first blood vessel video to the first reference frame image.

Each of the first blood vessel video and the second blood vessel video may include a planar blood vessel video including a plurality of planar frame images, and the method may further include generating a three-dimensional shape of the target blood vessel based on the determined correspondence relationship.

The obtaining of the first blood vessel video and the second blood vessel video may include confirming whether an electrocardiogram signal mapped to at least one blood vessel video of the first blood vessel video or the second blood vessel video exists, when the electrocardiogram signal mapped to the at least one blood vessel video exists, determining whether a phase of the electrocardiogram signal is analyzable, and when the phase of the electrocardiogram signal is analyzable, obtaining the at least one blood vessel video.

An electronic apparatus may include a video obtainment unit configured to obtain a first blood vessel video and a second blood vessel video by capturing a target blood vessel at a plurality of positions, and a processor configured to, for each of the first blood vessel video and the second blood vessel video, select, from among a plurality of frame images included in the corresponding blood vessel video, a reference frame image of the corresponding blood vessel video by using a score for analyzability of the target blood vessel shown in each frame image, and determine a correspondence relationship between the reference frame image of the first blood vessel video and the reference frame image of the second blood vessel video based on information associated with a cardiac cycle of each reference frame image.

## BRIEF DESCRIPTION OF DRAWINGS

FIG. 1 is a view illustrating a structure of a medical electronic apparatus according to an embodiment.

FIG. 2 is a view illustrating a change in a projection angle of radiation according to the rotation of a C-arm included in an electronic apparatus according to an embodiment.

FIG. 3 is a view illustrating a process in which an electronic apparatus, according to an embodiment, calculates a linear distance from a radiation source to a target blood vessel.

FIG. 4 is a flowchart illustrating an example of a method of processing a medical image, performed by an electronic apparatus, according to various embodiments.

FIG. 5 is a view illustrating an example of an operation in which an electronic apparatus, according to various embodiments, selects a reference frame image from among a plurality of frame images of a blood vessel video.

FIG. 6 is a view illustrating an example of an operation in which an electronic apparatus, according to various embodiments, verifies a candidate frame image.

FIG. 8 is a view illustrating an example of corresponding reference frame images determined by an electronic apparatus, according to various embodiments, based on a cardiac phase of a reference frame image.

FIG. 9 is a view illustrating an example of a correspondence relationship between reference frames of a plurality of blood vessel videos according to various embodiments.

FIG. 10 is a view illustrating an example configuration of an electronic apparatus according to various embodiments.

## BEST MODE FOR CARRYING OUT THE INVENTION

The following detailed structural or functional description is provided as an example only, and various alterations and modifications may be made to the examples. Accordingly, the embodiments are not construed as limited to the disclosure and should be understood to include all changes, equivalents, and replacements within the idea and the technical scope of the disclosure.

Terms, such as first, second, and the like, may be used herein to describe components. Each of these terminologies is not used to define an essence, order, or sequence of a corresponding component but used merely to distinguish the corresponding component from other component(s). For example, a first component may be referred to as a second component, and similarly, the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component.

As used herein, the singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like components and a repeated description related thereto will be omitted.

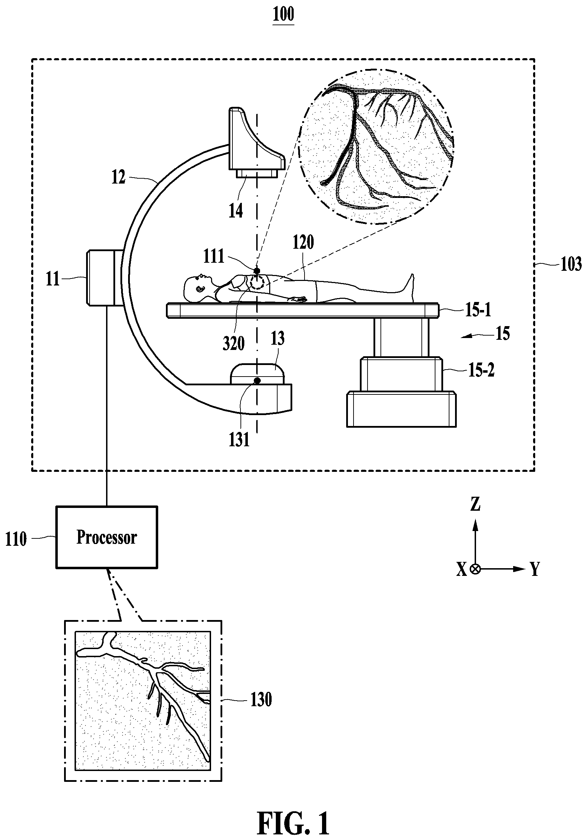

FIG. 1 is a view illustrating a structure of a medical electronic apparatus according to an embodiment.

A medical electronic apparatus 100 (hereinafter, referred to as an 'electronic apparatus'), according to an embodiment, may restore a three-dimensional shape of a target from a medical image. The medical image in which a blood vessel of a subject is captured may also be referred to as a blood vessel video. The electronic apparatus 100 may determine a correspondence relationship between frame images included in a plurality of blood vessel videos and/or restore a three-dimensional shape of a blood vessel, and may also be referred to as a medical image processing apparatus. The three-dimensional shape of the blood vessel restored by the electronic apparatus 100 may be used to calculate an index related to blood flow (e.g., quantitative flow ratio (QFR), fractional flow reserve (FFR)).

The electronic apparatus 100 may include a video obtainment unit and a processor 110. In the present specification, an example in which the video obtainment unit is an imaging device (e.g., a medical imaging apparatus) is mainly described, but the disclosure is not limited thereto, and the video obtainment unit may be a device that receives a medical image (e.g., a blood vessel video) from an external imaging device based on wired and/or wireless communication.

The video obtainment unit may include a device that captures a blood vessel video by irradiating radiation onto the blood vessel of a subject. Types of blood vessels may include, for example, the left main coronary artery (LM), the left anterior descending artery (LAD), the left circumflex artery (LCX), the right coronary artery (RCA), and the like. The video obtainment unit may capture a blood vessel using coronary angiography. For example, the video obtainment unit may include a body portion 11, a C-arm 12, an irradiator 13, and a radiation detector 14. An imaging device having the C-arm 12 may also be referred to as a C-arm imaging device.

The C-arm 12 may have a curved arc shape in a C-shape with one side open. For example, when the C-arm 12 is vertically erected with respect to a floor on which the electronic apparatus 100 is placed, the C-arm 12 may have a shape that is symmetrical with respect to the plane that is parallel to the floor and includes an isocenter 111. The isocenter 111 is a portion or point that serves as the center of the flux of radiation emitted from various positions despite the rotation of the C-arm, and may be defined as a point where a first rotation axis of the C-arm intersects with a second rotation axis thereof. The isocenter 111 may represent the center of a rotation trajectory of the irradiator 13 (or a radiation source (not shown)) generated according to the rotation of the C-arm 12. The C-arm 12 may have a shape that is open toward the isocenter 111.

The body portion 11 may be connected to the C-arm 12. The body portion 11 may be mechanically coupled to the C-arm 12. The C-arm 12 may rotate with respect to the body portion 11. The C-arm 12 may rotate within a YZ plane with respect to the body portion 11. For example, a protrusion included in the body portion 11 and a moving guide included in the C-arm 12 may be coupled to each other, and the C-arm 12 may rotate within the YZ plane along the moving guide with respect to a rotation axis that is parallel to an X-axis and passes through the isocenter 111. In addition, the C-arm 12 may also rotate within an XZ plane with respect to the body portion 11. For example, in a state in which a point, where the body portion 11 contacts the C-arm 12, is fixed, the C-arm 12 may rotate within the XZ plane with respect to a rotation axis that is parallel to a Y-axis and passes through the isocenter 111.

The irradiator 13 and the radiation detector 14 may be disposed on an inner surface of the C-arm 12 to face each other with the isocenter 111 therebetween. Each of the irradiator 13 and the radiation detector 14 may be connected to the C-arm 12. The irradiator 13 may include one or more radiation sources, and may emit radiation toward a subject through the one or more radiation sources. The radiation detector 14 may include a radiation detection sensor, and may detect radiation emitted from the irradiator 13 and transmitted through the blood vessel of a subject through the radiation detection sensor. Because the irradiator 13 and the radiation detector 14 are connected to each other through the C-arm 12 and rotate as a single unit, when the radiation detector 14 moves, the irradiator 13 may be positioned on the opposite side of the radiation detector 14 with respect to the isocenter 111. As will be described later, the electronic apparatus 100 may capture a medical image (e.g., a blood vessel video) at a plurality of capturing positions while changing a position (e.g., a capturing position) of the radiation detector 14.

A subject may be laid on a table 15. More specifically, the table 15 may include a table top 15-1 on which a subject may be laid and a table support 15-2 that supports the table top 15-1. The table support 15-2 may be fixed to the floor. For example, the table top 15-1 may be movably coupled to the table support 15-2. The electronic apparatus 100 may further include an actuator (not shown) that changes a position of the table top 15-1 with respect to the table support 15-2. The electronic apparatus 100 may move the table top 15-1 with respect to the table support 15-2 through the actuator (not shown). The actuator (not shown) may include a motor (e.g., an electric motor) and a power transmission structure.

The table top 15-1 may move in the lateral direction and the vertical direction with respect to the table support 15-2. For example, the electronic apparatus 100 may move the table top 15-1 in the lateral direction on the plane (e.g., an XY plane) parallel to the surface of the table. The surface of the table may represent an upper surface of the table top 15-1. The electronic apparatus 100 may also move the table top 15-1 in the vertical direction on an axis (e.g., a Z-axis) perpendicular to the plane (e.g., the XY plane) parallel to the surface of the table. In the following description, moving the table 15 may represent moving the table top 15-1 with respect to the table support 15-2. For example, the electronic apparatus 100 may move the table 15 in order to accurately irradiate radiation onto the target blood vessel of the subject.

The electronic apparatus 100 may control the C-arm 12 such that the C-arm 12 rotates around the subject positioned on the table 15. The electronic apparatus 100 may fix the C-arm 12 and then irradiate radiation onto the target blood vessel of the subject on the table 15 through the radiation source of the irradiator 13 connected to the C-arm 12. The radiation irradiated onto the blood vessel of the subject may be X-rays.

In an embodiment, the subject 120 may be positioned on the table top 15-1 of the electronic apparatus 100. The electronic apparatus 100 may generate the blood vessel video 130 in which the target blood vessel 320 of the subject 120 is captured using radiation emitted from the radiation source 131.

The processor 110 of the electronic apparatus 100 may derive quantitative analysis results, such as a diameter of the target blood vessel 320 and a length of a lesion, from the generated blood vessel video 130. In order to derive quantitative analysis results related to the target blood vessel 320 from the blood vessel video 130, the processor 110 may convert a distance measured in the blood vessel video 130 into a real-world physical distance, such as μm, mm, or cm. For example, the processor 110 may convert the distance measured in the blood vessel video 130 into the physical distance by using a calibration factor.

Specifically, the physical distance may be calculated by multiplying the calibration factor by the number of pixels corresponding to the distance measured in the blood vessel video 130. For example, the physical distance may be expressed as Equation 1 below.

[Equation 1]

$$\text{Physical distance} = \text{Calibration factor} \times \text{Number of pixels}$$

Referring to Equation 1, the calibration factor may represent a physical distance corresponding to a length (e.g., a horizontal length or a vertical length) of one pixel constituting the blood vessel video 130. In order to accurately calculate the physical distance, it is necessary to accurately calculate the calibration factor.

The calibration factor may represent a spatial relationship between an object in the blood vessel video and an object in the real world. The calibration factor may be calculated by multiplying an imager pixel spacing by a value obtained by dividing a source-to-object distance (SOD), which is a distance from the radiation source to a target object, by a source-to-image receptor distance (SID), which is a distance from the radiation source to an image receptor. The imager pixel spacing may represent a length (e.g., a horizontal length or a vertical length) of one pixel constituting the blood vessel video. That is, the calibration factor may be expressed as Equation 2 below.

[Equation 2]

$$\text{Calibration factor} = \frac{\text{SOD}}{\text{SID}} \times \text{Imager pixel spacing}$$

In Equation 2, SOD may represent a linear distance from the source (e.g., the radiation source) to an object to be captured, and SID may represent a linear distance from the source to the image receptor. The source-to-image receptor distance (SID) and the imager pixel spacing may be extracted from metadata of Digital Imaging and Communications in Medicine (DICOM). DICOM may represent a standard specification for storing and transmitting data related to images generated by a medical electronic apparatus.

In an embodiment, the electronic apparatus 100 may irradiate radiation onto the target blood vessel 320 of the subject 120 positioned on the table top 15-1 by using the radiation source 131 included in the irradiator 13. The electronic apparatus 100 may calculate a target distance from the radiation source 131 to the target blood vessel 320 based on a first perpendicular distance from the isocenter 111 to the table 15 and a second perpendicular distance from the target blood vessel 320 to the table 15. Here, the first perpendicular distance from the isocenter 111 to the table 15 may represent a perpendicular distance from the isocenter 111 to the upper surface of the table top 15-1. Similarly, the second perpendicular distance from the target blood vessel 320 to the table 15 may represent a perpendicular distance from the target blood vessel 320 to the upper surface of the table top 15-1.

More specifically, before irradiating radiation onto the target blood vessel 320 using the radiation source 131, the electronic apparatus 100 may adjust the position of the target blood vessel 320 through the table 15. The electronic apparatus 100 may move the table 15 such that the radiation source 131, the target blood vessel 320, and the isocenter 111 are arranged on a straight line. In an embodiment, the electronic apparatus 100 may cause the radiation source 131, the target blood vessel 320, and the isocenter 111 to be arranged on a straight line by moving the table top 15-1 only in the lateral direction on the XY plane. In another embodiment, the electronic apparatus 100 may cause the radiation source 131, the target blood vessel 320, and the isocenter 111 to be arranged on a straight line by moving the table top 15-1 both in the lateral direction on the XY plane and in the vertical direction on the Z-axis.

In an embodiment, when the position of the radiation source 131 changes according to the rotation of the C-arm 12, the table top 15-1 may be moved together such that the radiation source 131, the target blood vessel 320, and the isocenter 111 are arranged on a straight line.

In an embodiment, the electronic apparatus 100 may arrange the isocenter 111 on the same straight line as the radiation source 131 and the target blood vessel 320 by moving the table 15 in the lateral direction on the plane including the surface of the table. The height of the table 15 may be fixed, but is not limited thereto and may also be variable. For example, the electronic apparatus 100 may calculate the height of the target blood vessel 320 from the floor. The electronic apparatus 100 may calculate the height of the target blood vessel 320 from the floor by adding the height of the target blood vessel 320 from the surface of the table and the height of the table 15 from the floor. However, embodiments are not limited thereto, and the electronic apparatus 100 may also obtain the height from the table 15 (e.g., the surface of the table) to the target blood vessel 320. The electronic apparatus 100 may generate a plane that is parallel to the floor (or the XY plane) and includes a point corresponding to the target blood vessel 320, based on the position (e.g., the height) of the target blood vessel 320. The electronic apparatus 100 may calculate a point (an 'intersection point') where the plane, which is parallel to the floor and includes the target blood vessel 320, intersects with a linear axis, which connects the radiation source 131 to the isocenter 111. The electronic apparatus may move the table top 15-1 in the lateral direction on the XY plane such that the target blood vessel 320 is positioned at the calculated intersection point.

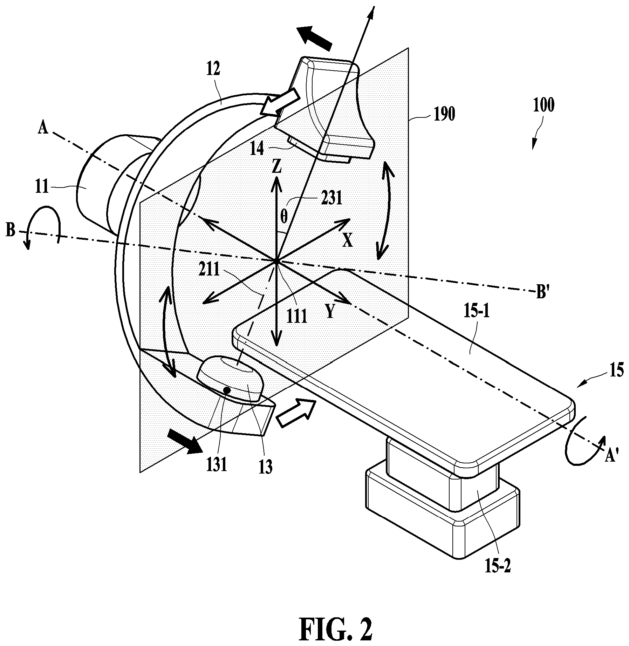

In an embodiment, after adjusting the position of the target blood vessel 320 through movement of the table top 15-1, the electronic apparatus 100 may calculate the target distance representing a linear distance from the radiation source 131 to the target blood vessel 320 using a plurality of parameters. The plurality of parameters may include, for example, the position of the table 15, the position of the target blood vessel 320, and the irradiation angle of radiation emitted from the radiation source 131 (e.g., the projection angle 231 of radiation in FIG. 2). More specifically, the electronic apparatus 100 may accurately calculate the target distance from the radiation source 131 to the target blood vessel 320 based on the first perpendicular distance from the isocenter 111 to the table 15, the second perpendicular distance from the target blood vessel 320 to the table 15, and the irradiation angle of radiation emitted from the radiation source 131.

FIG. 2 is a view illustrating a change in a projection angle of radiation according to the rotation of a C-arm included in an electronic apparatus according to an embodiment.

In an embodiment, the electronic apparatus 100 may irradiate radiation onto a target blood vessel of a subject positioned on the table 15 by using the radiation source 131 included in the irradiator 13.

The electronic apparatus 100, according to an embodiment, may rotate the radiation source based on at least one rotation axis among a first rotation axis A-A' that includes the isocenter 111 and is parallel to the table plane and a second rotation axis B-B' different from the first rotation axis A-A', based on the rotation of the C-arm. For example, the radiation source may be rotated in one of a plane that includes the isocenter 111 and is perpendicular to the first rotation axis A-A' parallel to the table plane, or a plane that includes the isocenter 111 and is perpendicular to the second rotation axis B-B' different from the first rotation axis A-A'. The second rotation axis B-B' may intersect with the first rotation axis A-A', and, for example, may be orthogonal to the first rotation axis A-A' at the isocenter 111. For reference, an example in which the C-arm and the radiation source rotate in one plane is described for convenience of explanation, but embodiments are not limited thereto, and the C-arm and the radiation source may also rotate with respect to two rotation axes A-A' and B-B' simultaneously.

For example, the electronic apparatus 100 may rotate the C-arm 12 about the first rotation axis A-A' with respect to the body portion 11. The first rotation axis A-A' may represent an axis connecting a point where the C-arm 12 is coupled to the body portion 11 to the isocenter 111. The position of the radiation source 131 may be changed according to the rotation of the C-arm 12. For example, according to the rotation of the C-arm 12 about the first rotation axis A-A', the radiation source 131 may be rotated while forming a rotation trajectory on the plane 190 that includes the isocenter 111 and is perpendicular to the first rotation axis A-A'. In FIG. 2, an X-Z plane is illustrated as the plane 190 perpendicular to the first rotation axis A-A' as an example.

The radiation source 131 may rotate while forming a rotation trajectory having a shape similar to a circle on the plane 190 according to the rotation of the C-arm 12. A rotation trajectory along which the radiation source 131 moves during the rotation of the C-arm 12 may not be geometrically a perfect circle, and may be a circle that is partially distorted due to deflection, vibration, or the like of the C-arm 12.

In addition, the electronic apparatus 100 may also rotate the C-arm 12 about the second rotation axis B-B' with respect to the body portion 11. The second rotation axis B-B' may be an axis that passes through the isocenter 111 and intersects with (e.g., is orthogonal to) the first rotation axis A-A'. For example, according to the rotation of the C-arm 12 about the second rotation axis B-B', the radiation source 131 may be rotated while forming a rotation trajectory on the plane that includes the isocenter 111 and is perpendicular to the second rotation axis B-B'. For reference, FIG. 2 illustrates an example in which the second rotation axis B-B' also rotates together while the C-arm rotates with respect to the first rotation axis A-A'. However, when the rotation of the C-arm 12 with respect to the second rotation axis B-B' occurs while the radiation source 131 and the radiation detector 14 are positioned on the Z-axis, the second rotation axis B-B' is parallel to the X-axis, and the radiation source 131 may be rotated while forming a rotation trajectory in the Y-Z plane.

When the position of the radiation source 131 is changed according to the rotation of the C-arm 12, the projection angle 231 (e.g., θ) of radiation emitted from the radiation source 131 may be changed. In other words, the projection angle 231 of radiation emitted from the radiation source may be changed according to the rotation of the C-arm 12.

The projection angle 231 of radiation may represent an angle between an axis (e.g., a Z-axis) orthogonal to the plane including the surface of the table 15 and the axis 211 corresponding to the irradiation direction of radiation emitted from the radiation source 131. The irradiation direction of radiation may represent a direction from the irradiator 13 (or the radiation source 131) toward the radiation detector 14 (or a radiation detection sensor). The axis 211 corresponding to the irradiation direction of radiation may represent an axis connecting the irradiator 13 to the radiation detector 14. The projection angle 231 of radiation may be an angle between -180 degrees and 180 degrees.

The isocenter 111 may be variously defined based on the rotation of the C-arm 12. For example, the isocenter 111 may represent a point where radiation irradiated from various positions of the radiation source according to the rotation of the C-arm 12 is intensively concentrated. In another example, the isocenter 111 may represent the geometric center of a rotation trajectory of the radiation source 131. In another example, the isocenter 111 may represent the rotation center of the C-arm 12.

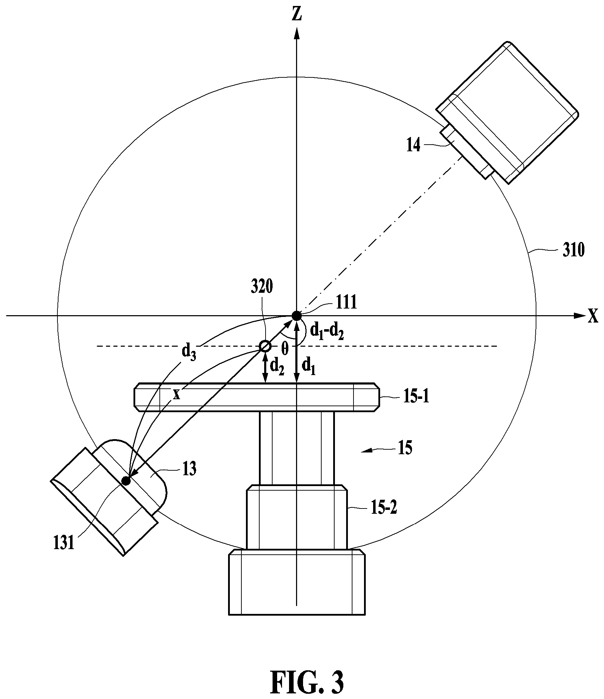

FIG. 3 is a view illustrating a process in which an electronic apparatus, according to an embodiment, calculates a linear distance from a radiation source to a target blood vessel.

FIG. 3 is a side view of an electronic apparatus (e.g., the electronic apparatus 100 of FIG. 1) as viewed from an XZ plane. Before irradiating radiation onto the target blood vessel 320 using the radiation source 131, the electronic apparatus may move the table top 15-1 to align the radiation source 131, the target blood vessel 320, and the isocenter 111 on a straight line. As described above, the position (e.g., the capturing position) of the radiation detector 14 may be moved according to the rotation of the C-arm 12. For example, the radiation detector 14 and the irradiator 13 may be moved in a region range 310 corresponding to a surface of a hemisphere according to the rotation of the C-arm 12.

In an embodiment, the electronic apparatus may calculate a first value $(d_1-d_2)$ by subtracting a second perpendicular distance, $d_2$, between a subject 120 (e.g., a target blood vessel) and the table 15 from a first perpendicular distance, $d_1$, between the isocenter 111 and the table 15. The first value $(d_1-d_2)$ may represent a perpendicular distance from the isocenter 111 to the target blood vessel 320. Then, the electronic apparatus may calculate a second value by dividing the calculated first value $(d_1-d_2)$ by a cosine value $(\cos(\theta))$ of the irradiation angle of radiation (e.g., the projection angle 231 of radiation in FIG. 2). The second value may represent a linear distance from the isocenter 111 to the target blood vessel 320. The electronic apparatus may calculate the target distance, x, from the radiation source 131 to the target blood vessel 320 as a value obtained by subtracting the calculated second value (i.e., the linear distance from the isocenter 111 to the target blood vessel 320) from a linear distance, $d_3$, between the radiation source 131 and the isocenter 111. For example, the target distance from the radiation source 131 to the target blood vessel 320 may be calculated as Equation 3 below.

[Equation 3]

$$SOD = Source\ to\ Isocenter - \frac{Isocenter\ to\ table - Object\ height}{\cos\theta}$$

In Equation 3 above, SOD may represent the linear distance from the radiation source 131 to the target blood vessel 320, Source to Isocenter may represent the linear distance $d_3$ from the radiation source 131 to the isocenter 111, *Isocenter to table* may represent the perpendicular distance $d_1$ from the isocenter 111 to the table 15, *Object height* may represent the perpendicular distance $d_2$ from the target blood vessel 320 to the table 15, and $\theta$ may represent the irradiation angle of radiation.

In an embodiment, after calculating the target distance, the electronic apparatus may calculate the calibration factor based on the calculated target distance. As described above, the electronic apparatus may convert a distance in the blood vessel video 130 into a physical distance by using the calculated calibration factor. As will be described later, in the present specification, the electronic apparatus may calculate a physical distance corresponding to the diameter of the target blood vessel by multiplying the calibration factor by the number of pixels corresponding to the diameter of the target blood vessel shown in a captured blood vessel video.

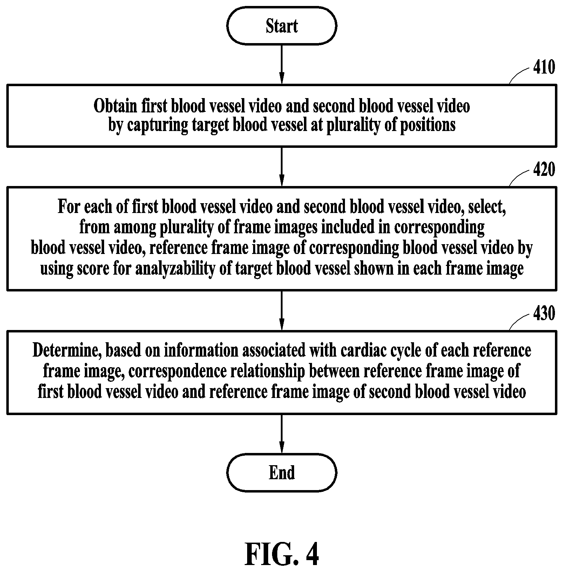

FIG. 4 is a flowchart illustrating an example of a method of processing a medical image, performed by an electronic apparatus, according to various embodiments.

The electronic apparatus, according to an embodiment, may determine a correspondence relationship between frame images included in a plurality of blood vessel videos.

In operation 410, the electronic apparatus obtains a first blood vessel video and a second blood vessel video by capturing the target blood vessel at a plurality of positions.

According to an embodiment, the target blood vessel may represent a major vessel. For example, the major vessel may be the left main coronary artery (LM), the right coronary artery (RCA), the left anterior descending artery (LAD), or the left circumflex artery (LCX).

A blood vessel video is a video in which the target blood vessel is captured, and may be a video in which other blood vessels, the lung, the diaphragm, the heart, a catheter inserted into a blood vessel, and the like are also included in addition to the target blood vessel. The blood vessel video may include a video captured for the target blood vessel during a certain time period. For example, the blood vessel video may include a plurality of frame images for the target blood vessel. The plurality of frame images included in one blood vessel video may be temporally adjacent. That the plurality of frame images is temporally adjacent may refer to that a capturing time of each frame image among the plurality of frame images is included within a threshold time length from a capturing time of another frame image.

A plurality of blood vessel videos may include videos in which the target blood vessel is captured at different angles from each other. For example, a plurality of blood vessel videos may be captured at a plurality of positions (e.g., capturing positions) through an irradiator and a radiation detector moved through the rotation of a C-arm of a C-arm imaging device.

For example, the electronic apparatus may load a plurality of blood vessel videos related to the target blood vessel stored in memory. In another example, the electronic apparatus may receive a plurality of blood vessel videos from an external device (e.g., an imaging device, a medical imaging apparatus) through a communicator. The external device (e.g., the C-arm imaging device, the medical imaging apparatus) may generate a plurality of blood vessel videos by capturing the target blood vessel at different angles, and may transmit the generated plurality of blood vessel videos to the electronic apparatus.

According to an embodiment, the electronic apparatus may obtain blood vessel videos when the blood vessel videos are analyzable based on electrocardiogram signals mapped to the blood vessel videos.

The electronic apparatus may confirm whether an electrocardiogram signal mapped to at least one blood vessel video of the first blood vessel video or the second blood vessel video exists. The electrocardiogram signal may refer to a signal recorded in a waveform by analyzing the electrical activity of the heart. The electrocardiogram signal may include signal values representing intensities of the electrocardiogram signal obtained at a plurality of timepoints. The electrocardiogram signal mapped to the blood vessel video may include a correspondence relationship between timepoints of the plurality of frame images of the blood vessel video and timepoints of signal values of the electrocardiogram signal. For example, the electrocardiogram signal mapped to the blood vessel video may include signal values of the electrocardiogram signal respectively corresponding to the plurality of frame images of the blood vessel video. When an electrocardiogram signal mapped to the blood vessel video does not exist, analysis of the blood vessel video (e.g., determination of a correspondence relationship) may be impossible, and thus the electronic apparatus may restrict obtaining of the blood vessel video.

When an electrocardiogram signal mapped to at least one blood vessel video exists, the electronic apparatus may determine whether the phase of the electrocardiogram signal is analyzable. The electronic apparatus may obtain a converted electrocardiogram signal by applying preprocessing to the electrocardiogram signal. The converted electrocardiogram signal may include the result of applying smoothing to the electrocardiogram signal. The smoothing may be performed by applying at least one of a Biosppy module or a Neurokit2 module, for example. The electronic apparatus may calculate the number of peaks per unit time in the converted electrocardiogram signal. The electronic apparatus may determine whether the phase of the electrocardiogram signal is analyzable based on the distribution of numbers of peaks per unit time in the converted electrocardiogram signal.

For example, when preprocessing is performed using the Biosppy module, when the ratio of numbers greater than or equal to a first threshold number among numbers of peaks per unit time of the converted electrocardiogram signal is greater than or equal to a first threshold ratio, it may be determined that the phase of the electrocardiogram signal is not analyzable. For example, when preprocessing is performed using the Neurokit2 module, when the ratio of numbers less than a second threshold number among numbers of peaks per unit time of the converted electrocardiogram signal is greater than or equal to a second threshold ratio, it may be determined that the phase of the electrocardiogram signal is not analyzable.

When the phase of the electrocardiogram signal is analyzable, the electronic apparatus may obtain at least one blood vessel video. As a result, the electronic apparatus may obtain blood vessel videos (e.g., the first blood vessel video, the second blood vessel video) to which an electrocardiogram signal having an analyzable phase is mapped.

In operation 420, for each of the first blood vessel video and the second blood vessel video, the electronic apparatus may select, from among a plurality of frame images included in the corresponding blood vessel video, a reference frame image of the corresponding blood vessel video by using a score for analyzability of the target blood vessel shown in each frame image.

The electronic apparatus may select, from among the plurality of frame images included in each blood vessel video, a reference frame image of the corresponding blood vessel video. The reference frame image may refer to an image selected as a frame image suitable for analyzing the target blood vessel (e.g., generating a three-dimensional shape of the target blood vessel) from among the plurality of frame images.

The electronic apparatus may select, from among the plurality of frame images of the first blood vessel video, a first reference frame image. The electronic apparatus may select, from among the plurality of frame images of the second blood vessel video, a second reference frame image. The electronic apparatus may select two or more frame images as reference frame images (e.g., the first reference frame image, the second reference frame image) from among the plurality of frame images of the blood vessel video (e.g., the first blood vessel video, the second blood vessel video). However, the electronic apparatus, according to various embodiments of the disclosure, is not limited to selecting two or more frame images as reference frame images, and may also select a single frame image as a reference frame image for at least one blood vessel video.

According to an embodiment, the electronic apparatus may calculate a score for each frame image included in the blood vessel video. The score for each frame image may represent a degree to which a portion corresponding to the target blood vessel shown in the corresponding frame image is suitable for analysis of the target blood vessel. Being suitable for analysis of the target blood vessel may refer to whether a region corresponding to the target blood vessel is clearly visible through the frame image. In various embodiments of the disclosure, that the target blood vessel is clearly visible in the frame image may refer to whether the blood vessel region corresponding to the target blood vessel in the frame image has a contrast greater than or equal to a threshold with other regions (e.g., the remaining regions) and/or a degree of sharpness of the blood vessel region shown in the frame image. For example, the score for each frame image may be determined based on at least one of information regarding blur of the corresponding frame image, information regarding a lesion shown in the corresponding frame image, or a concentration of a contrast medium corresponding to the corresponding frame image. In various embodiments of the disclosure, the score for the frame image may also be expressed as recommendation suitability and/or analysis suitability.

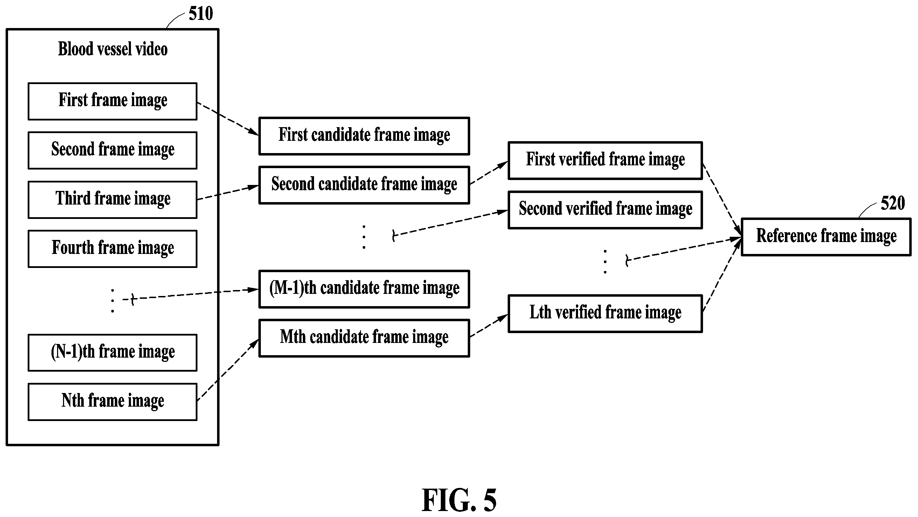

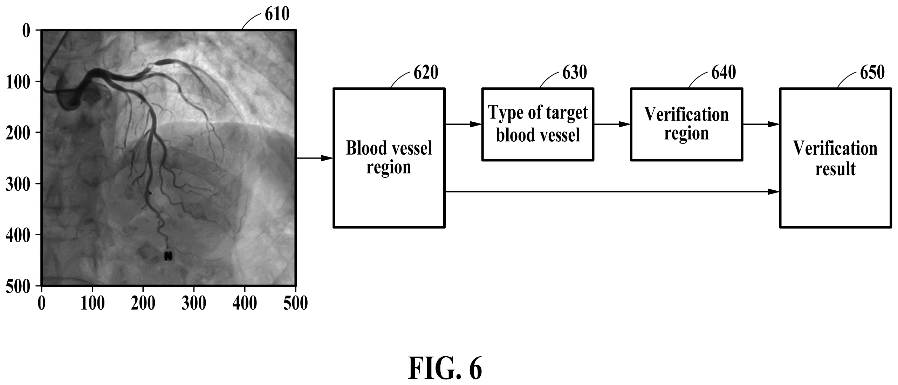

According to an embodiment, the score for each frame image may be calculated based on a score calculation model. The score calculation model may refer to a model generated and/or trained to output output data including a score for a frame image by being applied to input data corresponding to the frame image. The score calculation model may be built based on a machine learning model (e.g., a neural network, a convolution neural network). Calculation of the score for each frame image and selection of the reference frame image will be described in more detail later with reference to FIGS. 5 and 6.

In operation 430, the electronic apparatus may determine a correspondence relationship between the reference frame image of the first blood vessel video and a reference frame image of the second blood vessel video based on information associated with a cardiac cycle of each reference frame image.

Information associated with a cardiac cycle may include a cardiac phase. The cardiac phase of the reference frame image may refer to a phase of the reference frame image within a heartbeat cycle. For example, the cardiac phase of the reference frame image may refer to a phase of a signal value mapped to the reference frame image among a plurality of signal values of an electrocardiogram signal (ECG signal).

For example, the electrocardiogram signal may have periodicity according to the heartbeat cycle. The cardiac phase of the frame image (e.g., the reference frame image) may include a position of the signal value of the electrocardiogram signal corresponding to the frame image within the heartbeat cycle. One heartbeat cycle may include a P-wave and a QRS-complex wave, and may be defined as from a detection timepoint of a preceding P-wave (e.g., a start timepoint of the heartbeat cycle) to a detection timepoint of a subsequent P-wave (e.g., an end timepoint of the heartbeat cycle). The cardiac phase may be determined based on the ratio of a length from a start point of the heartbeat cycle to a timepoint corresponding to the signal value to the entire length of the heartbeat cycle, in one heartbeat cycle including the signal value of the electrocardiogram signal.

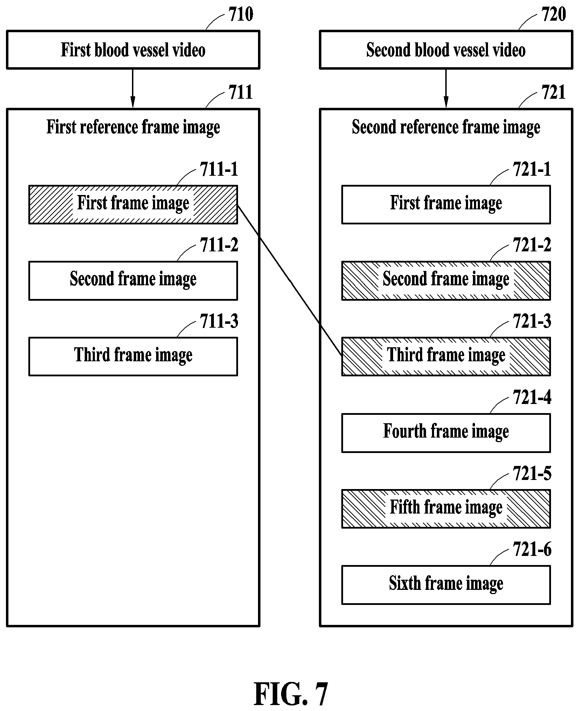

An operation of determining the correspondence relationship between reference frames based on the cardiac phase will be described in more detail with reference to FIG. 7.

When the correspondence relationship between the reference frame images of the plurality of blood vessel videos is determined, the electronic apparatus, according to an embodiment, may provide the electrocardiogram signal together with the reference frame images. For example, the electronic apparatus may determine that the first reference frame image (e.g., a single reference frame image) of the first blood vessel video corresponds to the second reference frame image (e.g., a single reference frame image) of the second blood vessel video. The electronic apparatus may display a waveform of the electrocardiogram signal mapped to the first blood vessel video together with the first reference frame image, and display a graphic representation indicating a signal value mapped to the first reference frame image in the displayed waveform. The electronic apparatus may display a waveform of the electrocardiogram signal mapped to the second blood vessel video together with the second reference frame image, and display a graphic representation indicating a signal value mapped to the second reference frame image in the displayed waveform.

The electronic apparatus, according to an embodiment, may generate a three-dimensional shape (e.g., a three-dimensional blood vessel video) of the target blood vessel by using the correspondence relationship. For example, each of the first blood vessel video and the second blood vessel video may include a planar blood vessel video (e.g., a two-dimensional blood vessel video) including a plurality of planar frame images (e.g., two-dimensional frame images). The electronic apparatus may determine the correspondence relationship between the reference frame images captured at different positions. The electronic apparatus may generate a three-dimensional shape of the target blood vessel based on the determined correspondence relationship.

FIG. 5 is a view illustrating an example of an operation in which an electronic apparatus, according to various embodiments, selects a reference frame image from among a plurality of frame images of a blood vessel video.

The electronic apparatus, according to an embodiment, may select, from among the plurality of frame images included in a blood vessel video 510, a reference frame image 520 for which a correspondence relationship with a frame image of another blood vessel video is to be determined.

The electronic apparatus may select the reference frame image 520 by using at least one of a score for analyzability of the frame image, the type of a target blood vessel shown in the frame image, or the physical size of the target blood vessel shown in the frame image.

The electronic apparatus, according to an embodiment, may select candidate frame images from among the plurality of frame images in order of highest scores based on the scores of the plurality of frame images. The electronic apparatus may select frame images having the highest scores among the plurality of frame images as the candidate frame images based on the scores of the plurality of frame images. The electronic apparatus may select a predetermined number of frame images as the candidate frame images. The candidate frame image may refer to a frame image that is selectable as a reference frame image (i.e., a candidate for the reference frame image).

The electronic apparatus may obtain the blood vessel video 510 including the plurality of frame images. For example, in FIG. 5, the blood vessel video 510 may include N frame images. Here, N may be an integer greater than or equal to 2. The electronic apparatus may calculate, for each of the plurality of frame images of the blood vessel video 510, a score representing analyzability of the target blood vessel shown in the corresponding frame image. The electronic apparatus may calculate a plurality of scores of the plurality of frame images by repeatedly applying the score calculation model to each frame image.

The electronic apparatus, according to an embodiment, may select, from among the plurality of frame images, candidate frame images as frame images in order of highest scores. The electronic apparatus may select a predetermined number of candidate frame images having the highest scores from among the plurality of frame images. For example, the electronic apparatus may select, from among the plurality of frame images, frame images in order of the highest scores. The number of frame images may be determined based on the number (e.g., N) of the plurality of frame images included in the blood vessel video 510, or may be determined as a constant. For example, in FIG. 5, the electronic apparatus may select M candidate frame images having the highest scores from among the plurality of frame images. Here, M may be an integer greater than or equal to 1 and less than or equal to N. The electronic apparatus, according to an embodiment, may set the number of the reference frame images of the first blood vessel video and the number of the reference frame images of the second blood vessel video differently from each other. For example, the electronic apparatus may set the number of the reference frame images of the second blood vessel video to be greater than the number of the reference frame images of the first blood vessel video.

According to an embodiment, the electronic apparatus may determine a score for analyzability of the target blood vessel based on a morphologic feature of the target blood vessel. The morphologic feature of the target blood vessel may include at least one of blur, panning, the concentration of a contrast medium, information regarding a lesion (e.g., visibility of a lesion), or the physical size of the target blood vessel.

The electronic apparatus may detect a region of interest (ROI) from each frame image based on information regarding a capturing angle of the corresponding frame image. The electronic apparatus may detect the region of interest based on a capturing angle of the blood vessel video (e.g., the projection angle 231 of radiation in FIG. 2). For example, when information regarding the capturing angle of the blood vessel video corresponds to an RCA-RAO view, the electronic apparatus may detect the region of interest based on a middle portion (e.g., mid) of the blood vessel. For example, when information regarding the capturing angle of the blood vessel video corresponds to an LM-Spider view, the electronic apparatus may detect the region of interest based on a proximal portion of the blood vessel.

The electronic apparatus may determine a score for a morphologic feature of the target blood vessel included in the region of interest of each frame image. The score calculation model, according to an embodiment, may be generated and/or trained to output a score representing a morphologic feature of the target blood vessel by being applied to an input image corresponding to the frame image.

The electronic apparatus, according to an embodiment, may obtain verified frame images by verifying the selected candidate frame images. The electronic apparatus may verify the candidate frame images based on the type of the target blood vessel. The verified frame image may refer to a frame image that has passed verification among the candidate frame images (e.g., a verified candidate frame image). Verification of a candidate frame image may be determined based on a position of a blood vessel region corresponding to the target blood vessel within the candidate frame image. For example, the candidate frame images may be verified based on whether the blood vessel region is located in (or overlaps with) a specific region or is not located in (or does not overlap with) a specific region. The electronic apparatus may perform verification based on the type of the target blood vessel shown in each candidate frame image. Verification of the candidate frame images and/or obtaining of the verified frame images will be described in more detail later with reference to FIG. 6.

When the electronic apparatus determines that the verification of a candidate frame image has failed, the electronic apparatus may restrict the corresponding candidate frame image from being selected as a verified frame image. When the electronic apparatus determines that the verification of a candidate frame image has succeeded, the electronic apparatus may select the corresponding candidate frame image as a verified frame image.

When the verification of at least one candidate frame image has failed (e.g., when at least one candidate frame image is not selected as a verified frame image), the electronic apparatus may add a frame image different from the plurality of candidate frame images among the plurality of frame images to the candidate frame images. Instead of a candidate frame image for which the verification has failed, the electronic apparatus may add another frame image among the plurality of frame images of the blood vessel video 510 to the candidate frame images. Another frame image may refer to an image that has never been selected as a candidate frame image.

For example, the electronic apparatus may add (e.g., supplement) another frame image(s) having highest score(s) among the frame images that have not been selected as candidate frame images among the plurality of frame images to the candidate frame images. As a result, based on the verification result, the electronic apparatus may supplement (e.g., replace) a candidate frame image that is not selected as a verified frame image with another frame image.

The electronic apparatus, according to an embodiment, may select a reference frame image 520 from among the verified frame images. For example, the electronic apparatus may select the reference frame image 520 based on the physical size of the target blood vessel shown in the verified frame images.

The electronic apparatus may determine the physical size of the target blood vessel shown in each verified frame image based on a blood vessel region corresponding to the target blood vessel segmented from the corresponding verified frame image and a calibration factor.

For example, the electronic apparatus may segment the blood vessel region corresponding to the target blood vessel from each of the verified frame images. The electronic apparatus may extract the blood vessel region from the verified frame image by using a blood vessel segmentation model. The blood vessel segmentation model may refer to a model generated and/or trained to output output data including a region corresponding to the target blood vessel in a frame image by being applied to input data corresponding to the frame image (e.g., a verified frame image). The blood vessel segmentation model may be built based on a machine learning model (e.g., a neural network, a convolution neural network). The electronic apparatus may extract a plurality of blood vessel regions of the verified frame images by repeatedly applying the blood vessel segmentation model to each verified frame image.

For example, after extracting the blood vessel region from the candidate frame image during a verification process of the candidate frame image, when the candidate frame image is selected as a verified frame image, the electronic apparatus may determine the physical size of the target blood vessel shown in the candidate frame image (or the verified frame image). The electronic apparatus may determine the physical size of the target blood vessel by using the blood vessel region extracted during the verification process of the candidate frame image. In other words, when the blood vessel region extracted during the verification process of the candidate frame image is available and the candidate frame image is selected as a verified frame image, the electronic apparatus may skip an operation of segmenting (or extracting) a blood vessel region in the operation of determining the physical size of the target blood vessel.

The electronic apparatus may determine the physical size (e.g., the area) of the target blood vessel shown in the corresponding candidate frame image based on the blood vessel region and the calibration factor. As described above with reference to FIG. 1, the calibration factor may represent the spatial relationship between the object in the blood vessel video 510 (e.g., the blood vessel region shown in the blood vessel video) and the object in the real world (e.g., the target blood vessel). For example, the electronic apparatus may calculate an area calibration factor corresponding to each pixel of each candidate frame image based on the calibration factor. The electronic apparatus may determine the physical size of the target blood vessel by multiplying the number of pixels included in the blood vessel region segmented from the candidate frame image by the area calibration factor.

The electronic apparatus may select the reference frame image 520 from among the verified frame images based on the physical size of the target blood vessel. The electronic apparatus may select the reference frame image 520 having the largest physical size(s) of the target blood vessel from among the verified frame images. According to an embodiment, the electronic apparatus may select a single reference frame image or a plurality of reference frame images from the blood vessel video 510.

FIG. 6 is a view illustrating an example of an operation in which an electronic apparatus, according to various embodiments, verifies a candidate frame image.

The electronic apparatus, according to an embodiment, may verify each candidate frame image 610 based on the type 630 of the target blood vessel shown in each of the candidate frame images.

The electronic apparatus may determine the type 630 of the target blood vessel shown in each candidate frame image 610 based on a blood vessel region 620 corresponding to the target blood vessel segmented from the corresponding candidate frame image 610.

For example, the electronic apparatus may segment the blood vessel region 620 corresponding to the target blood vessel from each of the candidate frame images. As described above with reference to FIG. 5, the electronic apparatus may extract the blood vessel region 620 from the candidate frame image 610 by using the blood vessel segmentation model.

The electronic apparatus may determine the type 630 of the target blood vessel shown in the candidate frame image 610 based on the blood vessel region 620. The electronic apparatus may determine the type 630 of the target blood vessel based on a blood vessel classification model. The blood vessel classification model may refer to a model generated and/or trained to output output data representing the type 630 of the target blood vessel by being applied to input data corresponding to a region corresponding to the target blood vessel (e.g., the blood vessel region 620). The output data of the blood vessel classification model may include a likelihood score for each of a plurality of candidate blood vessel types, and the electronic apparatus may determine a candidate blood vessel type having a maximum likelihood score among the plurality of likelihood scores as the type 630 of the target blood vessel. For example, the plurality of candidate blood vessel types may include at least one of the left main coronary artery (LM), the left anterior descending artery (LAD), the left circumflex artery (LCX), or the right coronary artery (RCA). The blood vessel classification model may be built based on a machine learning model (e.g., a neural network, a convolution neural network). The electronic apparatus may extract blood vessel types of the target blood vessel shown in each of the candidate frame images by repeatedly applying the blood vessel classification model to each candidate frame image 610.

The electronic apparatus may determine a verification result 650 of the candidate frame image based on a verification region 640 mapped to the type 630 of the target blood vessel and the blood vessel region 620. For example, when the verification region 640 mapped to the type 630 of the target blood vessel includes at least a portion of the blood vessel region 620, the electronic apparatus may restrict the corresponding candidate frame image 610 from being selected as verified frame images. The electronic apparatus may determine that the verification of the corresponding candidate frame image 610 has failed.

When the target blood vessel is detected in the verification region 640, the verification region 640 may represent a position at which the blood vessel region 620 is not suitable for analysis in the candidate frame image 610. For example, when the target blood vessel is detected in the verification region 640, the verification region 640 may refer to a region for detecting that only a portion of a part necessary for analysis of the target blood vessel is shown (e.g., the remaining part necessary for analysis of the target blood vessel is missing) in the candidate frame image 610. The verification region 640 may be set differently depending on the type of a blood vessel.

The verification regions 640 may correspond to a plurality of candidate blood vessel types. According to an embodiment, the verification regions 640 may individually correspond to a plurality of candidate blood vessel types. According to an embodiment, one verification region may correspond to at least one blood vessel type among the plurality of candidate blood vessel types, and another verification region may correspond to the remaining blood vessel type(s) among the plurality of candidate blood vessel types.

For example, a first verification region may correspond to a first candidate blood vessel type (e.g., the right coronary artery (RCA)). The first verification region may include pixels adjacent to a left side and an upper side of the candidate frame image 610. A second verification region may correspond to a second candidate blood vessel type (e.g., at least one of the left main coronary artery (LM), the left anterior descending artery (LAD), or the left circumflex artery (LCX)). The second verification region may include pixels adjacent to a portion of the left side and the upper side of the candidate frame image 610. The portion of the left side may refer to a portion extending upward from a lower end of the left side.

When the type 630 of the target blood vessel is the first candidate blood vessel type, the electronic apparatus may restrict the candidate frame image 610 from being selected as a verified frame image when an overlapping region between the first verification region and the blood vessel region 620 exists. For example, when the type 630 of the target blood vessel is the right coronary artery (RCA), the electronic apparatus may restrict the candidate frame image 610 from being selected as a verified frame image when at least one of pixels located on the left side or the upper side of the candidate frame image 610 is included in the blood vessel region 620.

When the type 630 of the target blood vessel is the second candidate blood vessel type, the electronic apparatus may exclude the candidate frame image 610 from the reference frame image when an overlapping region between the second verification region and the blood vessel region 620 exists. When the type 630 of the target blood vessel is different from the right coronary artery, the electronic apparatus may restrict the candidate frame image 610 from being selected as a verified frame image when at least one of pixels located on the portion of the left side or the upper side of the candidate frame image 610 is included in the blood vessel region 620.

In various embodiments of the disclosure, the electronic apparatus is mainly described as determining the type 630 of the target blood vessel based on the candidate frame image 610, but the disclosure is not limited thereto.

According to an embodiment, the electronic apparatus may determine the type of the target blood vessel based on the blood vessel video before selecting the candidate frame image 610 from among the plurality of frame images of the blood vessel video. For example, upon obtaining at least one blood vessel video of the first blood vessel video or the second blood vessel video, the electronic apparatus may determine the type of the target blood vessel shown in the obtained at least one blood vessel video. The electronic apparatus may determine the type of the target blood vessel shown in the blood vessel video based on at least one frame image among the plurality of frame images.

According to an embodiment, the electronic apparatus may obtain information regarding the type of the target blood vessel. For example, the electronic apparatus may obtain an input of a user designating the type of the target blood vessel. The electronic apparatus may perform verification of the candidate frame image based on the type of the target blood vessel designated by the input of the user.

FIG. 7 is a view illustrating an operation in which an electronic apparatus, according to various embodiments, determines a correspondence relationship between reference frame images.

The electronic apparatus, according to an embodiment, may determine the correspondence relationship between reference frame images selected from the plurality of blood vessel videos.

The electronic apparatus may select a correspondence candidate of a first reference frame image 711 of a first blood vessel video 710 from among the second reference frame images. The electronic apparatus may select the correspondence candidate of the first reference frame image 711 based on the physical size of the target blood vessel shown in the second reference frame images of a second blood vessel video 720.

According to an embodiment, a first frame image 711-1, a second frame image 711-2, and a third frame image 711-3 may be selected as the first reference frame images 711 from among the plurality of frame images of the first blood vessel video 710. The electronic apparatus may select, for each of the first reference frame images, the correspondence candidate from among the second reference frame images and determine the correspondence relationship independently from each other. According to an embodiment, the correspondence candidates of the first reference frame images may be the same, different, at least partially overlapping, or not overlapping at all. In addition, the second reference frame images to which the first reference frame images correspond may be the same or different from each other. For example, the first frame image 711-1 and the second frame image 711-2 may correspond to the same third frame image 721-3 among the second reference frame images 721.

Hereinafter, an example of the operation of selecting the second reference frame image 721 corresponding to each of the first reference frame image 711 (e.g., the first frame image 711-1) will be mainly described.

For example, the electronic apparatus may obtain a first physical size of the target blood vessel shown in the first reference frame image 711 (e.g., the first frame image 711-1) of the first blood vessel video 710. The electronic apparatus may obtain second physical sizes of the target blood vessel shown in each of the second reference frame images (e.g., a first frame image 721-1, a second frame image 721-2, the third frame image 721-3, a fourth frame image 721-4, a fifth frame image 721-5, and a sixth frame image 721-6) of the second blood vessel video 720.

According to an embodiment, the physical size (e.g., the first physical size, the second physical size) of the target blood vessel shown in the reference frame image (e.g., the first reference frame image 711, the second reference frame image 721) may be already determined (e.g., calculated) during the selection process of the reference frame image as described above with reference to FIG. 5. When the physical size determined during the selection process of the reference frame image is available, the electronic apparatus may skip an operation of determining (or calculating) the physical size of the target blood vessel shown in the reference frame image in an operation of determining a correspondence relationship.

The electronic apparatus may select the correspondence candidate of the first reference frame image 711 from among the second reference frame images based on the first physical size and the second physical size. For each of the second reference frame images, when the ratio of the second physical size of the corresponding second reference frame image 721 to the first physical size is less than a threshold ratio (e.g., 0.8), the electronic apparatus may exclude the corresponding second reference frame image 721 from the correspondence candidate. When the ratio of the second physical size of the corresponding second reference frame image 721 to the first physical size is greater than or equal to the threshold ratio, the electronic apparatus may select the corresponding second reference frame image 721 as the correspondence candidate.

For example, the electronic apparatus may select the second reference frame image 721 having a second physical size greater than or equal to a value obtained by multiplying the first physical size by a predetermined threshold multiple (e.g., 0.8 times) as the correspondence candidate of the first reference frame image 711. In FIG. 7, For example, the electronic apparatus may select the second frame image 721-2, the third frame image 721-3, and the fifth frame image 721-5 as the correspondence candidates of the first frame image 711-1.

When selecting the correspondence candidate of the first reference frame image 711 based on the first physical size results in excluding all of the second reference frame images from the correspondence candidates of the first reference frame image 711, the electronic apparatus, according to an embodiment, may select all of the second reference frame images as the correspondence candidates. For example, for all of the second reference frame images, when the ratio of each second physical size to the first physical size is less than a threshold ratio, the electronic apparatus may select all of the second reference frame images as the correspondence candidates of the first reference frame image 711.

For example, when all of the second physical sizes are less than a value obtained by multiplying the first physical size by a predetermined threshold multiple (e.g., 0.8 times), the electronic apparatus may determine all of the second reference frame images (e.g., the first frame image 721-1, the second frame image 721-2, the third frame image 721-3, the fourth frame image 721-4, the fifth frame image 721-5, and the sixth frame image 721-6) as the correspondence candidates of the first reference frame image 711.

The electronic apparatus may match, from among the selected correspondence candidates of the first reference frame image 711, the second reference frame image 721 having a cardiac phase closest to the cardiac phase (e.g., the phase of the electrocardiogram signal) of the first reference frame image 711 to the first reference frame image 711.

For example, the electronic apparatus may obtain a P-value representing the cardiac phase (e.g., the phase of the electrocardiogram signal) of the first reference frame image 711. For example, the P-value may be determined based on the ratio of a length from the start point of the heartbeat cycle to the timepoint corresponding to the signal value to the entire length of the heartbeat cycle. However, in various embodiments of the disclosure, the P-value is not limited to being determined based on the start point of the heartbeat cycle, and may be determined based on an arbitrary point (e.g., the center point of the heartbeat cycle) of the heartbeat cycle. The electronic apparatus may obtain P-values representing phases of electrocardiogram signals mapped to the correspondence candidates (e.g., the second frame image 721-2, the third frame image 721-3, and the fourth frame image 721-4). The electronic apparatus may determine that the third frame image 721-3, which is the second reference frame image 721 having a P-value with the smallest difference from a P-value of the first reference frame image 711 (e.g., the first frame image 711-1) among the correspondence candidates, corresponds to the first reference frame image 711. In other words, the electronic apparatus may determine that the first frame image 711-1 among the first reference frame images 711 corresponds to the third frame image 721-3 among the second reference frame images 721.

When a score of the second reference frame image 721 having the closest cardiac phase (e.g., the phase of the electrocardiogram signal) is less than a threshold score, the electronic apparatus, according to an embodiment, may cancel selection of the first reference frame image 711. When a score for analyzability of the second reference frame image 721 corresponding to the first reference frame image 711 is less than or equal to a threshold score, the electronic apparatus may cancel selection of the first reference frame image 711. For example, when a score for analyzability of the third frame image 721-3 selected as corresponding to the first frame image 711-1 is less than a threshold score, the electronic apparatus may cancel selection of the first frame image 711-1 as the first reference frame image 711.

The electronic apparatus may add at least one frame image to the first reference frame image 711 from among the plurality of frame images of the first blood vessel video 710. The electronic apparatus may add another frame image, which has not been selected as the first reference frame image 711, to the first reference frame image 711 from among the plurality of frame images of the first blood vessel video 710. The electronic apparatus may select another frame image to be added to the first reference frame image 711 according to the operations described above with reference to FIGS. 4 to 6.

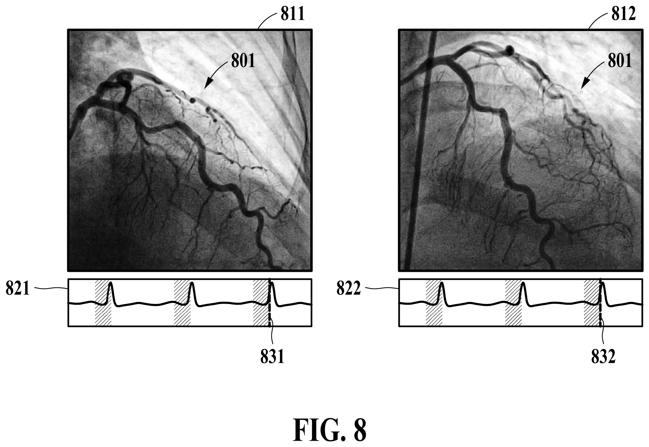

FIG. 8 is a view illustrating an example of corresponding reference frame images determined by an electronic apparatus, according to various embodiments, based on a cardiac phase of a reference frame image.

The electronic apparatus, according to an embodiment, may determine that a first reference frame image obtained from a first blood vessel video corresponds to a second reference frame image obtained from a second blood vessel video.

In an embodiment, the electronic apparatus may obtain a planar blood vessel video and an electrocardiogram signal mapped to the planar blood vessel video. For example, an external device may record the electrocardiogram signal by detecting electrical activity of the heart simultaneously with capturing the planar blood vessel video. The external device may transmit the planar blood vessel video and the electrocardiogram signal mapped to the corresponding planar blood vessel video to the electronic apparatus.

In an embodiment, the electronic apparatus may select the first reference frame image 811 from the first blood vessel video. The electronic apparatus may determine the cardiac phase (or the phase of the electrocardiogram signal) corresponding to the first reference frame image 811. For example, the first reference frame image 811 may correspond to the timepoint 831 in a QRS interval of the electrocardiogram signal 821 corresponding to the first blood vessel video. The electronic apparatus may select the second reference frame image 812 corresponding to the first reference frame image based on the phase of the electrocardiogram signal 821 of the first reference frame image 811. More specifically, the electronic apparatus may select the second reference frame image 812 corresponding to the timepoint 832 at which a phase identical to that of the electrocardiogram signal of the first reference frame image 811 appears, by using the electrocardiogram signal 822 corresponding to the second blood vessel video.

Because positions and/or shapes of blood vessels included in the target blood vessel 801 are continuously changed due to the heartbeat of a subject, the electronic apparatus may determine the correspondence relationship between reference frame images 811 and 812, of which the phases of the electrocardiogram signals match, within a plurality of planar blood vessel videos (e.g., the first blood vessel video and the second blood vessel video). The electronic apparatus may generate a three-dimensional shape of the target blood vessel by using the corresponding reference frame images 811 and 812, and may thereby reduce an error in the three-dimensional shape of the target blood vessel due to the change in a position of the target blood vessel according to the heartbeat.

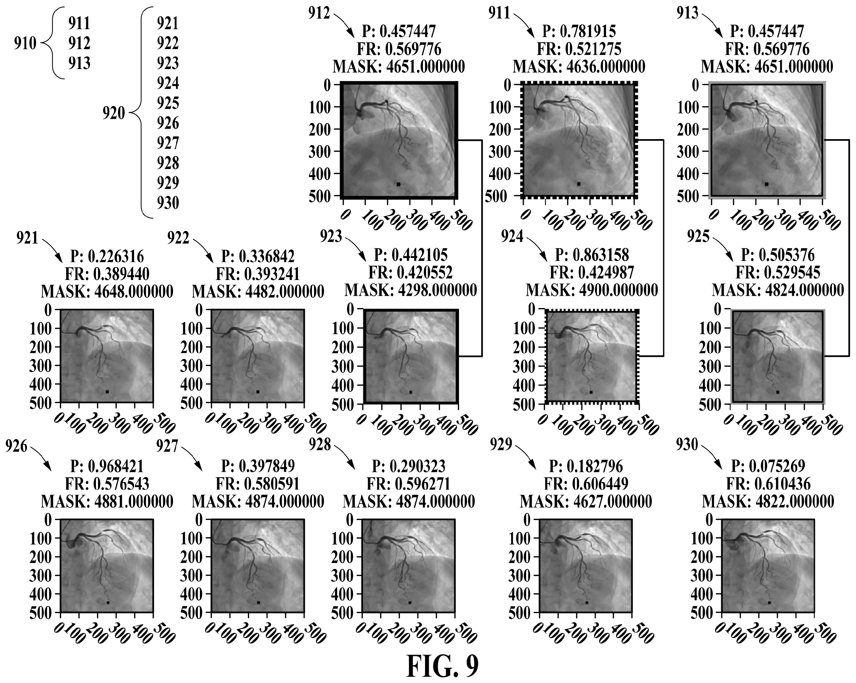

FIG. 9 is a view illustrating an example of a correspondence relationship between reference frames of a plurality of blood vessel videos according to various embodiments.

According to an embodiment, a first reference frame image 910 of the first blood vessel video may include three frame images (e.g., a first frame image 911, a second frame image 912, and a third frame image 913). A second reference frame image 920 of the second blood vessel video may include ten frame images (e.g., a first frame image 921, a second frame image 922, a third frame image 923, a fourth frame image 924, a fifth frame image 925, a sixth frame image 926, a seventh frame image 927, an eighth frame image 928, a ninth frame image 929, and a tenth frame image 930). The electronic apparatus may determine, among the first reference frame images 910, the second reference frame image 920 corresponding to the first frame image 911 as the fourth frame image 924. The electronic apparatus may determine, among the first reference frame images 910, the second reference frame image 920 corresponding to the second frame image 912 as the third frame image 923. The electronic apparatus may determine, among the first reference frame images 910, the second reference frame image 920 corresponding to the third frame image 913 as the fifth frame image 925.

For each of the first reference frame image(s) 910, when the electronic apparatus determines the second reference frame image 920 to which the corresponding first reference frame image 910 corresponds, the electronic apparatus may complete the determination of the correspondence relationship. The electronic apparatus may obtain a first correspondence relationship between the first frame image 911 and the fourth frame image 924, a second correspondence relationship between the second frame image 912 and the third frame image 923, and a third correspondence relationship between the third frame image 913 and the fifth frame image 925, as the correspondence relationship between reference frame images.

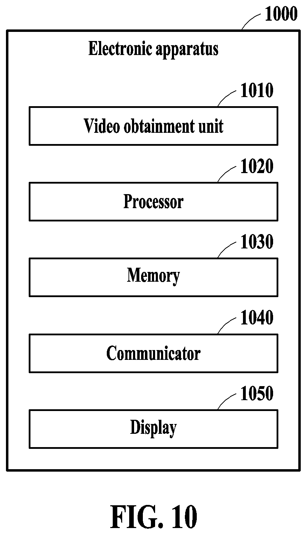

FIG. 10 is a view illustrating an example configuration of an electronic apparatus according to various embodiments.

An electronic apparatus 1000, according to an embodiment, may include a video obtainment unit 1010, a processor 1020, memory 1030, a communicator 1040, and a display 1050.

The video obtainment unit 1010 may obtain a plurality of blood vessel videos related to the target blood vessel. For example, the video obtainment unit 1010 may be integrally implemented with the communicator 1040 to obtain blood vessel videos related to the target blood vessel from an external device communicatively connected to the electronic apparatus 1000. In another example, the video obtainment unit 1010 may be implemented as an imaging device to obtain video data captured for the target blood vessel, and may obtain a blood vessel video by processing the video data.

The processor 1020 may obtain a plurality of blood vessel videos through the video obtainment unit 1010. The processor 1020 may select, for each of a plurality of blood vessel videos, a reference frame image of the corresponding blood vessel video. The processor 1020 may determine a correspondence relationship between the reference frame images based on a cardiac phase of the reference frame image.

The memory 1030 may store information regarding a blood vessel video, a plurality of frame images, a reference frame image, a cardiac phase, and/or a correspondence relationship. The memory 1030 may store instructions for obtaining a blood vessel video, selecting a reference frame image, and/or determining a correspondence relationship.

The communicator 1040 may transmit at least one of a blood vessel video, a reference frame image, a cardiac phase, and/or a correspondence relationship. The communicator 1040 may establish a wired communication channel and/or a wireless communication channel with an external device (e.g., an imaging device, another electronic device 1000, or a server), and may, for example, establish communication through cellular communication, short-range wireless communication, local area network (LAN) communication, Bluetooth, wireless fidelity (Wi-Fi) direct or infrared data association (IrDA), or a long-range communication network such as a legacy cellular network, a fourth generation (4G) and/or fifth generation (5G) network, next generation communication, the Internet, or a computer network (e.g., LAN or WAN).

The display 1050 may visualize at least one of a blood vessel video, a reference frame image, a cardiac phase, an

**EP 4 765 152 A1**

electrocardiogram signal, and/or a correspondence relationship.

The embodiments described herein may be implemented using a hardware component, a software component, and/or a combination thereof. A processing device may be implemented using one or more general-purpose or special-purpose computers, such as, for example, a processor, a controller and an arithmetic logic unit (ALU), a digital signal processor (DSP), a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording media.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter.

The above-described devices may be configured to act as one or more software modules in order to perform the operations of the above-described examples, or vice versa.

As used herein, "A or B", "at least one of A and B", "at least one of A or B", "A, B or C", "at least one of A, B and C", and "at least one of A, B, or C," each of which may include any one of the items listed together in the corresponding one of the phrases, or all possible combinations thereof.

Although the example embodiments have been described with reference to the limited drawings, one of ordinary skill in the art may apply various technical modifications and variations based thereon. For example, suitable results may be achieved if the described techniques are performed in a different order, and/or if components in a described system, structure, device, or circuit are combined in a different manner, and/or replaced or supplemented by other components or their equivalents.

Therefore, other implementations, other examples, and equivalents to the claims are also within the scope of the following claims.

**Claims**

1. A method of processing a medical image, performed by an electronic apparatus, the method comprising:

    obtaining a first blood vessel video and a second blood vessel video by capturing a target blood vessel at a plurality of positions;
    for each of the first blood vessel video and the second blood vessel video, selecting, from among a plurality of frame images included in the corresponding blood vessel video, a reference frame image of the corresponding blood vessel video by using a score for analyzability of the target blood vessel shown in each frame image; and
    determining a correspondence relationship between the reference frame image of the first blood vessel video and the reference frame image of the second blood vessel video based on information associated with a cardiac cycle of each reference frame image.

2. The method of claim 1, wherein the selecting of the reference frame image comprises:

selecting candidate frame images from among the plurality of frame images in order of highest scores based on scores of the plurality of frame images;
obtaining verified frame images by verifying the selected candidate frame images based on a type of the target blood vessel; and
selecting the reference frame image from among the verified frame images.

3. The method of claim 2, wherein the selecting of the candidate frame images comprises:

detecting a region of interest (ROI) from each frame image based on information regarding a capturing angle of the corresponding frame image; and
determining the score for a morphologic feature of the target blood vessel included in the region of interest of each frame image.

4. The method of claim 2, further comprising:
upon obtaining at least one blood vessel video of the first blood vessel video or the second blood vessel video, determining a type of the target blood vessel shown in the obtained at least one blood vessel video.

5. The method of claim 2,
wherein the obtaining of the verified frame images comprises:

determining a type of the target blood vessel shown in each candidate frame image based on a blood vessel region corresponding to the target blood vessel segmented from the corresponding candidate frame image; and
when a verification region mapped to the type of the target blood vessel includes at least a portion of the blood vessel region, restricting the corresponding candidate frame image from being selected as the verified frame images.

6. The method of claim 2,
wherein the selecting of the reference frame image from among the verified frame images comprises:

determining a physical size of the target blood vessel shown in each verified frame image based on a blood vessel region corresponding to the target blood vessel segmented from the corresponding verified frame image and a calibration factor; and
selecting a reference frame image from among the verified frame images based on the physical size of the target blood vessel.

7. The method of claim 1,
wherein the determining of the correspondence relationship comprises:

selecting, based on a physical size of the target blood vessel shown in second reference frame images of the second blood vessel video, a correspondence candidate of a first reference frame image of the first blood vessel video from among the second reference frame images; and
matching, from among the selected correspondence candidates, a second reference frame image having a cardiac phase closest to a cardiac phase of the first reference frame image to the first reference frame image.

8. The method of claim 7,
wherein the selecting of the correspondence candidate comprises:

for each of the second reference frame images, when a ratio of a second physical size of the target blood vessel shown in the corresponding second reference frame image to a first physical size of the target blood vessel shown in the first reference frame image is less than a threshold ratio, excluding the corresponding second reference frame image from the correspondence candidate; and
when the ratio of the physical size of the target blood vessel shown in the corresponding second reference frame image to the first physical size is greater than or equal to the threshold ratio, selecting the corresponding second reference frame image as the correspondence candidate.

9. The method of claim 7,
wherein the selecting of the correspondence candidate comprises:
for all of the second reference frame images, when a ratio of each second physical size to a first physical size of the

# EP 4 765 152 A1

target blood vessel shown in the first reference frame image is less than a threshold ratio, selecting all of the second reference frame images as correspondence candidates.

10. The method of claim 7,
   wherein the matching comprises:

   when a score of the second reference frame image having the closest cardiac phase is less than a threshold score, canceling selection of the first reference frame image; and
   adding at least one frame image from among the plurality of frame images of the first blood vessel video to the first reference frame image.

11. The method of claim 1,

   wherein each of the first blood vessel video and the second blood vessel video comprises a planar blood vessel video including a plurality of planar frame images, and
   the method further comprises generating a three-dimensional shape of the target blood vessel based on the determined correspondence relationship.

12. The method of claim 1,
   wherein the obtaining of the first blood vessel video and the second blood vessel video comprises:

   confirming whether an electrocardiogram signal mapped to at least one blood vessel video of the first blood vessel video or the second blood vessel video exists;
   when the electrocardiogram signal mapped to the at least one blood vessel video exists, determining whether a phase of the electrocardiogram signal is analyzable; and
   when the phase of the electrocardiogram signal is analyzable, obtaining the at least one blood vessel video.

13. A computer-readable recording medium storing one or more computer programs comprising instructions for performing the method of claim 1.

14. An electronic apparatus comprising:

   a video obtainment unit configured to obtain a first blood vessel video and a second blood vessel video by capturing a target blood vessel at a plurality of positions; and
   a processor configured to, for each of the first blood vessel video and the second blood vessel video, select, from among a plurality of frame images included in the corresponding blood vessel video, a reference frame image of the corresponding blood vessel video by using a score for analyzability of the target blood vessel shown in each frame image, and
   determine a correspondence relationship between the reference frame image of the first blood vessel video and the reference frame image of the second blood vessel video based on information associated with a cardiac cycle of each reference frame image.

**FIG. 1**

**FIG. 2**

**FIG. 3**

Start

410

Obtain first blood vessel video and second blood vessel video
by capturing target blood vessel at plurality of positions

420

For each of first blood vessel video and second blood vessel video, select,
from among plurality of frame images included in corresponding
blood vessel video, reference frame image of corresponding blood vessel video by
using score for analyzability of target blood vessel shown in each frame image

430

Determine, based on information associated with cardiac cycle of each reference
frame image, correspondence relationship between reference frame image of
first blood vessel video and reference frame image of second blood vessel video

End

# FIG. 4

FIG. 5

FIG. 6

**FIG. 7**

**FIG. 8**

FIG. 9

1000

Electronic apparatus

1010

Video obtainment unit

1020

Processor

1030

Memory

1040

Communicator

1050

Display

# FIG. 10

**EP 4 765 152 A1**

## INTERNATIONAL SEARCH REPORT

| | International application No. |
|---|---|
| | **PCT/KR2024/012101** |

| A. | CLASSIFICATION OF SUBJECT MATTER |
|---|---|

**G16H 30/40**(2018.01)i; **G16H 30/20**(2018.01)i; **A61B 5/00**(2006.01)i; **A61B 5/353**(2021.01)i; **G06V 10/25**(2022.01)i; **G06V 10/46**(2022.01)i; **G06V 10/764**(2022.01)i; **G06T 7/11**(2017.01)i; **G06T 7/62**(2017.01)i

According to International Patent Classification (IPC) or to both national classification and IPC

| B. | FIELDS SEARCHED |
|---|---|

Minimum documentation searched (classification system followed by classification symbols)

G16H 30/40(2018.01); A61B 5/00(2006.01); A61B 6/03(2006.01); G06T 7/11(2017.01); G06T 7/30(2017.01); G16H 50/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 의료 영상(medical image), 매칭(matching), 혈관(blood vessel), 프레임 이미지 (frame image)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2542972 B1 (THE ASAN FOUNDATION et al.) 15 June 2023 (2023-06-15)<br>See paragraphs [0003]-[0058] and figure 1. | 1-3,7,10-14 |
| Y | | 4-6,8-9 |
| Y | GALASSI, Francesca et al. 3D reconstruction of coronary arteries from 2D angiographic projections using nonuniform rational basis splines (NURBS) for accurate modelling of coronary stenoses. PLOS ONE. vol. 13, no. 1, pp. 1-23, 03 January 2018. Retrieved from < https://doi.org/10.1371/journal.pone.0190650>.<br>See pages 3-12. | 4-6,8-9 |
| A | KR 10-2021-0108178 A (FOUNDATION OF SOONGSIL UNIVERSITY-INDUSTRY COOPERATION) 02 September 2021 (2021-09-02)<br>See claims 1-11 and figure 7. | 1-14 |
| A | KR 10-2021-0016860 A (THE ASAN FOUNDATION et al.) 17 February 2021 (2021-02-17)<br>See claims 1-7 and figure 3. | 1-14 |

| ✓ Further documents are listed in the continuation of Box C. | ✓ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 November 2024** | **21 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

EP 4 765 152 A1

**INTERNATIONAL SEARCH REPORT**

International application No.

**PCT/KR2024/012101**

**C.    DOCUMENTS CONSIDERED TO BE RELEVANT**

| Category* | Citation of document, with indication, where appropriate, of the relevant passages | Relevant to claim No. |
|---|---|---|
| A | JP 2019-088792 A (CATHWORKS LTD.) 13 June 2019 (2019-06-13)<br>See claims 1-34 and figure 9. | 1-14 |

Form PCT/ISA/210 (second sheet) (July 2022)

## INTERNATIONAL SEARCH REPORT
### Information on patent family members

International application No.

**PCT/KR2024/012101**

| Patent document cited in search report | | | Publication date (day/month/year) | Patent family member(s) | | | Publication date (day/month/year) |
|---|---|---|---|---|---|---|---|
| KR | 10-2542972 | B1 | 15 June 2023 | None | | | |
| KR | 10-2021-0108178 | A | 02 September 2021 | KR | 10-2347029 | B1 | 04 January 2022 |
| KR | 10-2021-0016860 | A | 17 February 2021 | KR | 10-2343889 | B1 | 30 December 2021 |
| | | | | US | 2022-0296205 | A1 | 22 September 2022 |
| | | | | WO | 2021-025461 | A1 | 11 February 2021 |
| JP | 2019-088792 | A | 13 June 2019 | CN | 104364446 | A | 18 February 2015 |
| | | | | CN | 104364446 | B | 09 November 2016 |
| | | | | CN | 105190630 | A | 23 December 2015 |
| | | | | EP | 3753494 | A1 | 23 December 2020 |
| | | | | EP | 3954298 | A2 | 16 February 2022 |
| | | | | IL | 239961 | B | 31 July 2019 |
| | | | | IL | 245180 | A | 30 June 2016 |
| | | | | JP | 2019-193808 | A | 07 November 2019 |
| | | | | JP | 2021-045558 | A | 25 March 2021 |
| | | | | JP | 6790179 | B2 | 25 November 2020 |
| | | | | JP | 7039442 | B2 | 22 March 2022 |
| | | | | KR | 10-2015-0110609 | A | 02 October 2015 |
| | | | | US | 11728037 | B2 | 15 August 2023 |
| | | | | US | 11816837 | B2 | 14 November 2023 |
| | | | | US | 2023-0084748 | A1 | 16 March 2023 |
| | | | | US | 2023-0282365 | A1 | 07 September 2023 |
| | | | | WO | 2014-111930 | A1 | 24 July 2014 |
| | | | | WO | 2015-059706 | A2 | 30 April 2015 |
| | | | | WO | 2015-059706 | A3 | 27 August 2015 |

Form PCT/ISA/210 (patent family annex) (July 2022)